# EUROPEAN PATENT SPECIFICATION

(11) **EP 4 212 038 B1**
(45) Date of publication and mention of the grant of the patent: **16.09.2026**
(21) Application number: 20965880.6
(22) Date of filing: 15.12.2020
(51) Int. Cl.: A24F 40/50, H02J 1/10, H02J 7/00

(54) **INHALATION DEVICE, CONTROL METHOD, AND PROGRAM**
INHALATIONSVORRICHTUNG, STEUERUNGSVERFAHREN UND PROGRAMM
DISPOSITIF D'INHALATION, PROCÉDÉ DE COMMANDE ET PROGRAMME

(43) Date of publication of application: 19.07.2023
(73) Proprietor: Japan Tobacco Inc., Tokyo 105-6927 (JP)
(72) Inventor: ONO, Yasuhiro, Tokyo 130-8603 (JP)
(74) Representative: Hoffmann Eitle
(86) International application number: PCT/JP2020/046703
(87) International publication number: WO 2022/130492

(56) References cited:
- WO-A1-2020/064347
- JP-A- 2019 509 022
- JP-A- 2020 058 394
- JP-A- 2020 527 944
- US-A1- 2017 215 477
- US-A1- 2019 274 354
- US-A1- 2020 237 005

## Description

### Technical Field

The present invention relates to an inhaler device, a control method, and a program.

### Background Art

Inhaler devices for generating material to be inhaled by users, such as electronic cigarettes and nebulizers, are widely used. For example, an inhaler device generates an aerosol containing a flavor component by using a substrate including, for example, an aerosol source for generating an aerosol and a flavor source for imparting the flavor component to the generated aerosol. The user inhales the aerosol containing the flavor component, generated by the inhaler device, so that the user can taste the flavor.

In recent years, studies have been conducted to use various kinds of power sources in inhaler devices. For example, Patent Literature 1 listed below discusses the use of a battery such as a lithium-ion battery or solid-state battery in an inhaler device.

US2017215477A1 discloses an aerosol-generating device including at least one electric heater and a first power supply configured to supply electrical energy only to the at least one electric heater. The aerosol-generating device also includes a controller configured to control the supply of electrical energy from the first power supply to the at least one electric heater and a second power supply configured to supply electrical energy to the controller.

WO2020064347A1 discloses an electronic cigarette having an electrical heater for heating an aerosol forming substrate to generate an inhalable aerosol. Control circuitry is provided to control the supply of electrical power to the electrical heater. A first battery has a first operating voltage, when fully charged, which is above a first threshold, and the first battery supplies electrical power to the control circuitry when in use. A second battery has a second operating voltage, when fully charged, which is below the first threshold, and the second battery supplies electrical power to the electrical heater, when in use.

US2020237005A1 an aerosol generation device including: a power supplier comprising a first battery and a second battery; a controller; and a heater, wherein the controller is configured to control the power supplier to operate according to one of a first mode in which power is supplied to the heater by using the first battery and a second mode in which power is supplied to the heater by using the second battery, and control the power supplier to supply greater power in the first mode than in the second mode.

### Citation List

### Patent Literature

Patent Literature 1: JP 2020-504599 A

### Summary of Invention

### Technical Problem

However, because the technologies disclosed in, for example, Patent Literature 1 were developed recently, there is yet room for improvement in many aspects.

The present invention has been made in consideration of the problem described above, and an object of the present invention is to provide a solution for more suitably using batteries in an inhaler device.

### Solution to Problem

The object is achieved by the subject matter of the independent claims. Further advantageous embodiments are defined by the dependent claims. Examples are provided to facilitate the understanding of the present disclosure. According to an example, there is provided an inhaler device including a generator configured to generate an aerosol using a substrate containing an aerosol source, a controller configured to control operation of the generator, and a first battery and a second battery that are discrete. The controller is configured to control the first battery or the second battery to supply electric power to the generator.

The controller may be configured to control the second battery to supply electric power to the controller and also control the first battery or the second battery to supply electric power to the generator.

The first battery is operable to supply electric power to the generator, and the second battery is operable to supply electric power to the controller and the generator.

The first battery may be an all-solid-state battery.

The first battery may be an all-solid-state battery produced by a stacking technique.

The output voltage of the first battery may be higher than 4.2 V.

The first battery may be positioned close to the generator.

The inhaler device may further include a temperature sensor configured to sense the temperature of the first battery and the temperature of the generator.

The controller may be configured to, depending on the temperature sensed by the temperature sensor, control the first battery or the second battery to supply electric power to the generator.

The controller may be configured to, when the temperature sensed by the temperature sensor satisfies a predetermined condition, control the first battery to supply electric power to the generator.

The controller may be configured to, when the inhaler device is coupled to an external power supply, provide switching control to cause only the first battery or both of the first battery and the second battery to be charged, depending on the temperature sensed by the temperature sensor.

The controller may be configured to, when the inhaler device is coupled to an external power supply, cause the first battery and the second battery to be charged.

The controller may be configured to, depending on the residual capacity of the first battery, provide switching control to cause the first battery or the second battery to supply electric power to the generator.

The controller may be configured to control the second battery to supply electric power to the first battery to charge the first battery.

The inhaler device may include a protection element configured to stop overcurrent. The protection element may be provided in a circuit coupling the first battery and the generator.

It is possible that the protection element is not provided in a circuit coupling the second battery and the controller.

The inhaler device may further include a switch provided in a circuit coupling the first battery and the generator. The switch may be configured to control connection and disconnection between the first battery and the generator.

The generator may be configured to generate the aerosol by heating the substrate.

To address the problem described above, according to another aspect of the present invention, there is provided a control method for controlling an inhaler device. The inhaler device includes a generator configured to generate an aerosol using a substrate containing an aerosol source, a controller configured to control operation of the generator, and a first battery and a second battery that are discrete. The control method includes, with respect to each of the generator and the controller, controlling the first battery or the second battery to supply electric power.

To address the problem described above, according to another aspect of the present invention, there is provided a program configured to be run by a computer for controlling an inhaler device. The inhaler device includes a generator configured to generate an aerosol using a substrate containing an aerosol source, a controller configured to control operation of the generator, and a first battery and a second battery that are discrete. The program causes the computer to execute a process including, with respect to each of the generator and the controller, controlling the first battery or the second battery to supply electric power.

### Advantageous Effects of Invention

As described above, the present invention provides a solution for more suitably using batteries in an inhaler device.

### Brief Description of Drawings

[Fig. 1] Fig. 1 is a schematic diagram of an inhaler device according to a first configuration example.
[Fig. 2] Fig. 2 is a schematic diagram of an inhaler device according to a second configuration example.
[Fig. 3] Fig. 3 is a block diagram illustrating an example of a detailed configuration of an inhaler device according to an embodiment.
[Fig. 4] Fig. 4 is a flowchart illustrating an example of a process flow implemented by the inhaler device according to the embodiment.

### Description of Embodiments

Hereinafter, a preferred embodiment of the present invention will be described in detail with reference to the accompanying drawings. It should be noted that in this specification and accompanying drawings, structural elements having substantially the same functional configurations are assigned the same reference numerals, and redundant descriptions thereof are not repeated.

In this specification and accompanying drawings, structural elements having substantially the same functional configurations are sometimes assigned the same reference numerals followed by different alphabets so that the structural elements are distinguished. For example, structural elements having substantially the same functional configurations are referred to as inhaler devices 100A and 100B when necessary. When it is unnecessary to distinguish structural elements having substantially the same functional configurations, only the same reference numerals are assigned. For example, when it is unnecessary to distinguish the inhaler devices 100A and 100B, it is simply referred to as an inhaler device 100.

### 1. Configuration example of inhaler device

An inhaler device generates material to be inhaled by a user. In the example described below, the material generated by the inhaler device is an aerosol. Alternatively, the material generated by the inhaler device may be gas.

### (1) First configuration example

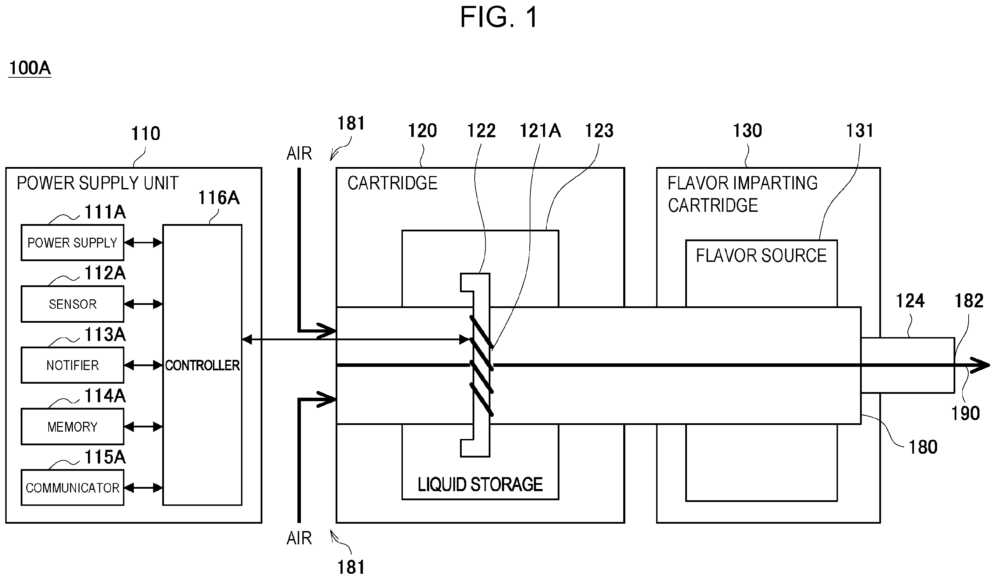

Fig. 1 is a schematic diagram of the inhaler device according to the first configuration example. As illustrated in Fig. 1, an inhaler device 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The power supply 111A stores electric power. The power supply 111A supplies electric power to the structural elements of the inhaler device 100A under the control of the controller 116A. The power supply 111A may be a rechargeable battery such as a lithium ion secondary battery.

The sensor 112A acquires various items of information regarding the inhaler device 100A. In an example, the sensor 112A may be a pressure sensor such as a microphone condenser, a flow sensor, or a temperature sensor, and acquire a value generated in accordance with the user's inhalation. In another example, the sensor 112A may be an input device that receives information input by the user, such as a button or a switch.

The notifier 113A provides information to the user. The notifier 113A may be a light-emitting device that emits light, a display device that displays an image, a sound output device that outputs sound, or a vibration device that vibrates.

The memory 114A stores various items of information for operation of the inhaler device 100A. The memory 114A may be a non-volatile storage medium such as flash memory.

The communicator 115A is a communication interface capable of communication in conformity with any wired or wireless communication standard. Such a communication standard may be, for example, Wi-Fi (registered trademark) or Bluetooth (registered trademark).

The controller 116A functions as an arithmetic processing unit and a control circuit, and controls the overall operations of the inhaler device 100A in accordance with various programs. The controller 116 is implemented by, for example, a central processing unit (CPU) and an electronic circuit such as a microprocessor.

The liquid storage 123 stores an aerosol source. The aerosol source is atomized to generate an aerosol. Examples of the aerosol source include liquids, for example polyhydric alcohols such as glycerine and propylene glycol, and water. The aerosol source may include a flavor component that is either derived from tobacco or not derived from tobacco. For the inhaler device 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1, the heater 121A is formed as a coil, wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. Subsequently, the supply of the electric power may be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is on the airflow path 180 at an upstream position (closer to the air inlet hole 181), and the flavor source 131 is on the airflow path 180 at a downstream position (closer to the air outlet hole 182). Air flowing in through the air inlet hole 181 when the user inhales mixes with the aerosol generated by the heater 121A. Subsequently, as indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is to be held in a mouth of the user during inhalation. The mouthpiece 124 has the air outlet hole 182. When the user inhales with the mouthpiece 124 in his/her mouth, the mixture fluid of the aerosol and the air enters the oral cavity of the user.

The configuration example of the inhaler device 100A has been described above. The inhaler device 100A is not limited to the above configuration, and may be configured in various ways as exemplified below.

In an example, the inhaler device 100A does not have to include the flavor imparting cartridge 130. In this case, the cartridge 120 includes the mouthpiece 124.

In another example, the inhaler device 100A may include various types of aerosol sources. Still another type of aerosol may be generated by mixing a plurality of types of aerosols generated from the plurality of types of aerosol sources in the airflow path 180 and causing a chemical reaction.

In addition, means for atomizing the aerosol source is not limited to heating by the heater 121A. For example, the means for atomizing the aerosol source may be vibration atomization or induction heating.

### (2) Second configuration example

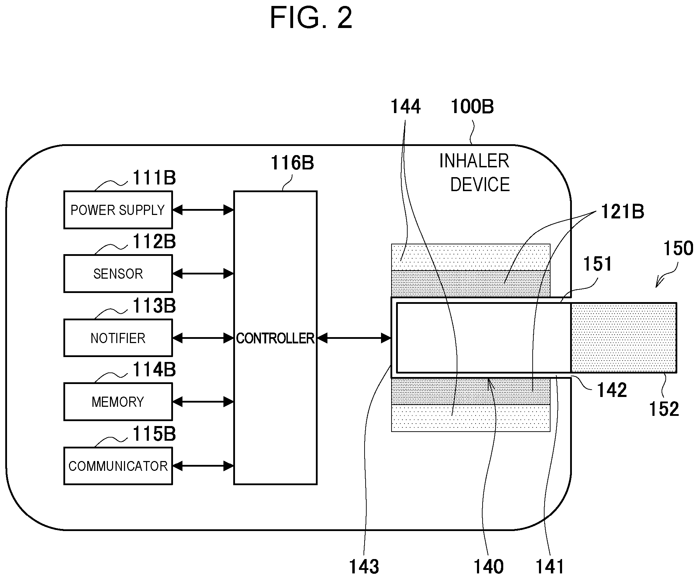

Fig. 2 is a schematic diagram of the inhaler device according to the second configuration example. As illustrated in Fig. 2, an inhaler device 100B according to the present configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the respective corresponding structural elements included in the inhaler device 100A according to the first configuration example.

The holder 140 has an internal space 141, and holds a stick substrate 150 in a manner partially accommodated in the internal space 141. The holder 140 has an opening 142 that allows the internal space 141 to communicate with outside. The holder 140 holds the stick substrate 150 that is inserted into the internal space 141 through the opening 142. For example, the holder 140 may be a tubular body having the opening 142 and a bottom 143 on its ends, and may define the pillar-shaped internal space 141. The holder 140 also has a function of defining the flow path of air to be supplied to the stick substrate 150. The air inlet hole that is an inlet of air to the flow path is provided, for example, at the bottom 143. The air outlet hole that is the outlet of air from the flow path is the opening 142.

The stick substrate 150 includes a substrate 151 and an inhalation port 152. The substrate 151 includes an aerosol source. In the present configuration example, the aerosol source is not limited to liquid and may be solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 from an air inlet hole (not illustrated), and the air and an aerosol generated from the substrate 151 reach inside the mouth of the user.

The heater 121B is configured in the same manner as the heater 121A according to the first configuration example, except that in the example illustrated in Fig. 2, the heater 121B is shaped as a film and disposed covering the outer circumference of the holder 40. Subsequently, heat produced from the heater 121B heats the substrate 151 of the stick substrate 150 from the outer circumference, generating the aerosol.

The heat insulator 144 prevents heat from transferring from the heater 121B to the other structural elements. For example, the heat insulator 144 may be a vacuum heat insulator or an aerogel heat insulator.

The configuration example of the inhaler device 100B has been described above. The inhaler device 100B is not limited to the above configuration, and may be configured in various ways as exemplified below.

In an example, the heater 121B may have a blade-like shape, and may be disposed so that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed so that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may sandwich the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the inhaler device 100B may also include the heater 121A, the liquid guide 122, the liquid storage 123, and the airflow path 180 according to the first configuration example. The air outlet hole 182 of the airflow path 180 may also function as an air inlet hole to the internal space 141. In this case, a mixture fluid of the air and an aerosol generated by the heater 121A flows into the internal space 141, mixes further with an aerosol generated by the heater 121B, and then reaches the oral cavity of the user.

### 2. Technical feature

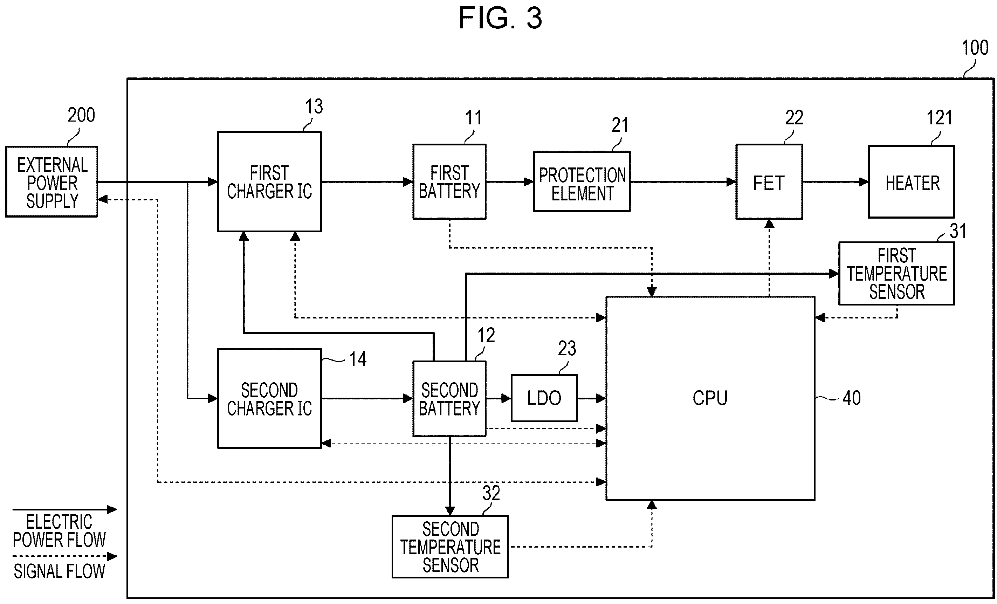

Fig. 3 is a block diagram illustrating an example of a detailed configuration of an inhaler device 100 according to the present embodiment. As illustrated in Fig. 3, the inhaler device 100 includes a first battery 11, a second battery 12, a first charger integrated circuit (IC) 13, a second charger IC 14, a protection element 21, a field effect transistor (FET) 22, a low dropout (LDO) 23, a first temperature sensor 31, a second temperature sensor 32, a CPU 40, and a heater 121. In Fig. 3, solid-line arrows indicate the flow of electric power. Dashed-line arrows indicate the flow of control signal.

The inhaler device 100 according to the present embodiment may be configured based on any configuration example of the first configuration example and the second configuration example described above. The first battery 11 and the second battery 12 illustrated in Fig. 3 are an example of the power supply 111 illustrated in Figs. 1 and 2. The first charger IC 13, the second charger IC 14, and the CPU 40 are an example of the controller 116 illustrated in Figs. 1 and 2. The first temperature sensor 31 and the second temperature sensor 32 are an example of the sensor 112 illustrated in Figs. 1 and 2. In the following, the user's action of inhaling an aerosol generated by the inhaler device 100 is also simply referred to as "inhalation" or "puff". The following describes the individual structural elements.

The heater 121 is an example of a generator for generating an aerosol to be inhaled by a user, using a substrate containing an aerosol source. Specifically, the heater 121 heats the substrate to generate the aerosol. The cartridge 120 and the flavor imparting cartridge 130 in the first configuration example and the stick substrate 150 in the second configuration example are examples of the substrate in the present embodiment. The inhaler device 100 uses the substrate attached to the inhaler device 100 to generate the aerosol. In the first configuration example, the cartridge 120 and the flavor imparting cartridge 130 connected to the power supply unit 110 are an example of the substrate attached to the inhaler device 100. In the second configuration example, the stick substrate 150 inserted in the inhaler device 100 is an example of the substrate attached to the inhaler device 100.

The first battery 11 and the second battery 12 are discrete batteries. As used herein, discrete means supplying electric power to different receivers. In other words, discrete means not supplying electric power to the same receiver at the same time. The first battery 11 and the second battery 12 are typically implemented by chargeable/dischargeable secondary batteries.

The first battery 11 may be an all-solid-state battery. An all-solid-state battery is a battery using a solid electrolyte in which ions are transferred between the anode and the cathode. It is known that because the electrolyte of all-solid-state batteries is solid, all-solid-state batteries have various advantages as compared to batteries using liquid electrolytes. Examples of the advantages include shape flexibility, high operational tolerance to high and low temperatures, and rapid charge/discharge capability. As a result of implementing the first battery 11 by an all-solid-state battery, the first battery 11 enjoys these advantages.

As illustrated in Fig. 3, the first battery 11 is coupled to the heater 121 via the protection element 21 and the FET 22. Thus, the first battery 11 is operable to supply electric power to the heater 121.

The first battery 11 may be an all-solid-state battery. As compared with a lithium-ion battery, an all-solid-state battery has shape flexibility; and the electrolyte is solid, which is unlikely to cause battery leakage. Hence, in one example, an all-solid-state battery is formed in a structure of a stack of many battery cells by using a stacking technique, so that high voltage is obtained. The first battery 11 may be an all-solid-state battery produced by a stacking technique. In this case, the first battery 11 supplies high output voltage, which is advantageous in heating by the heater 121. Moreover, because high output voltage is achieved by stacking, for example, when high-power voltage supply to the heater 121 is required, it is unnecessary to additionally provide a boost circuit. As a result, the power supply line from the first battery 11 to the heater 121 is made shorter. This eliminates power consumption by the pattern resistance in the power supply line from the first battery 11 to the heater 121 and also eliminates power loss due to voltage conversion by a boost circuit. Thus, the first battery 11 lasts longer.

It is desirable that the output voltage of the first battery 11 be higher than 4.2 V Considering that the voltage of lithium-ion battery per cell is 4.2 V at a maximum, with the structure described above, it is possible to output a voltage higher than lithium-ion batteries.

The first battery 11 also has a function of detecting voltage. The first battery 11 transmits a control signal representing information indicating the detected voltage to the CPU 40.

The second battery 12 may be a lithium-ion battery. Alternatively, the second battery 12 may be an all-solid-state battery. As illustrated in Fig. 3, the second battery 12 is coupled to the CPU 40 via the LDO 23. Thus, the second battery 12 is operable to supply electric power to the CPU 40. The second battery 12 is also coupled to the first temperature sensor 31, the second temperature sensor 32, and the first charger IC 13 and operable to supply electric power to the first temperature sensor 31, the second temperature sensor 32, and the first charger IC 13. The second battery 12 also has a function of detecting voltage. The second battery 12 transmits a control signal representing information indicating the detected voltage to the CPU 40.

The inhaler device 100 is coupleable to an external power supply 200. The external power supply 200 is a device for supplying electric power to the inhaler device 100. For example, a Universal Serial Bus (USB) is used to couple the inhaler device 100 and the external power supply 200. Between the inhaler device 100 and the external power supply 200, electric power or a control signal is provided or received via the USB. When the inhaler device 100 is coupled to the external power supply 200, at least either the first battery 11 or the second battery 12 is to be charged.

The first charger IC 13 controls charging of the first battery 11 based on a control signal from the CPU 40. Specifically, when the first charger IC 13 is coupled to the external power supply 200, and the first charger IC 13 receives from the CPU 40 a control signal for providing an instruction to charge the first battery 11, the first charger IC 13 supplies to the first battery 11 electric power supplied by the external power supply 200. As a result, the first battery 11 is charged.

The second charger IC 14 controls charging of the second battery 12 based on a control signal from the CPU 40. Specifically, when the second charger IC 14 is coupled to the external power supply 200, and the second charger IC 14 receives from the CPU 40 a control signal for providing an instruction to charge the second battery 12, the second charger IC 14 supplies to the second battery 12 electric power supplied by the external power supply 200. As a result, the second battery 12 is charged.

The charge configuration of the present embodiment described above is merely an example, and the present invention is not limited to this example. For example, the first charger IC 13 and the second charger IC 14 may be formed as one IC. In this case, the CPU 40 selects a battery to be charged. Which battery priority is given to for charging may be determined based on, for example, the detected battery voltage. Alternatively, priority may be given to a battery supplying electric power to the CPU 40.

The first battery 11 is operable to supply electric power to the heater 121. The second battery 12 is operable to supply electric power to the CPU 40. To increase the temperature of the heater 121 to a desired high temperature, it is necessary to provide large current to the heater 121. In this regard, because the first battery 11 implemented by a solid-state battery supplies electric power to the heater 121, it is possible to increase the temperature of the heater 121 to a desired temperature with a simple electrical circuit configuration. Incidentally, on the assumption that loads on the inhaler device 100 can be increased, countermeasures to abnormality are necessary. In this regard, with this configuration, the heater 121 and the CPU 40 are separable at the power-source level. As a result, when electric power is supplied to the heater 121, the stability of the CPU 40 is secured at the power-source level. With the help of the stable operation of the CPU 40 being continuously maintained, the operation of the inhaler device 100 is constantly monitored with a protection function controlled by the CPU 40. As such, the protection function is implemented in a stable manner.

The protection element 21 is a circuit for stopping overcurrent. In one example, the protection element 21 is implemented by a fuse, which is a non-restorable element, configured to be melted by Joule heat generated from overcurrent. As illustrated in Fig. 3, the protection element 21 is provided in a circuit coupling the first battery 11 and the heater 121. This configuration provides protection for the heater 121 that needs large current. The protection element 21 is not provided in a circuit coupling the second battery 12 and the CPU 40. Because the protection element 21 is provided in only the circuit related to the heater 121, unlike the case in which the protection element 21 is also provided in the circuit related to the CPU 40, while protection is provided for the heater 121 that needs large current, electric power to the CPU 40 is not blocked when the protection element 21 operates. Accordingly, if some abnormality occurs in the heater 121, the CPU 40 operates in a normal manner, the abnormality can be detected by the CPU 40 and another detector element, and additionally, a notification about the occurrence of abnormality can be provided for the user. This improves the safety of the entire device.

The FET 22 is a switch provided in a circuit coupling the first battery 11 and the heater 121, configured to control connection and disconnection between the first battery 11 and the heater 121. The FET 22 controls connection and disconnection between the first battery 11 and the heater 121 based on a control signal from the CPU 40. When the FET 22 connects the first battery 11 to the heater 121, the first battery 11 supplies electric power to the heater 121. When the FET 22 disconnects the first battery 11 from the heater 121, the first battery 11 does not supply electric power to the heater 121.

The LDO 23 is a circuit provided between the second battery 12 and the CPU 40, configured to convert the voltage supplied by the battery 12 into a voltage usable by the CPU 40 to supply stable voltage with reduced noise. This configuration makes the voltage supplied to the CPU 40 stable, thereby achieving stable operation of the CPU 40.

The first temperature sensor 31 senses the temperature of the first battery 11 and the temperature of the heater 121. In one example, the first temperature sensor 31 is implemented by a thermistor, which measures temperature based on changes in electrical resistance with changes in temperature. The first temperature sensor 31 transmits a control signal representing information indicating a sensed temperature to the CPU 40.

The first temperature sensor 31 is positioned within the range in which the temperature of the first battery 11 and the temperature of the heater 121 can be sensed. Specifically, the first temperature sensor 31 is positioned close to the first battery 11 and the heater 121, for example adjacent to both of the first battery 11 and the heater 121. This configuration eliminates the need of providing a plurality of temperature sensors for sensing the temperature of the first battery 11 and the temperature of the heater 121.

In relation to the positioning of the first temperature sensor 31, the first battery 11 is positioned close to the heater 121. For example, the first battery 11 is positioned adjacent to the heater 121 or adjacent to the heater 121 across the first temperature sensor 31. Such an arrangement is possible because the first battery 11 implemented by an all-solid-state battery has high operational tolerance to high temperatures. Further, although the first battery 11 can produce heat by fast charging, this arrangement reduces the effect of heat produced by fast charging on other structural elements. This is because the portion close to the heater 121 is engineered on the assumption that the heater 121 can produce heat; for example, the heat insulator 144 is provided. Moreover, because the first battery 11 is positioned close to the heater 121, the length of the power supply line from the first battery 11 to the heater 121 is reduced. This reduces power loss in the power supply line from the first battery 11 to the heater 121, and as a result, the first battery 11 lasts longer.

The second temperature sensor 32 senses the temperature of the second battery 12. In one example, the second temperature sensor 32 is implemented by a thermistor. The second temperature sensor 32 transmits a control signal representing information indicating the sensed temperature to the CPU 40. The second temperature sensor 32 is positioned within the range in which the temperature of the second battery 12 can be sensed. Specifically, the second temperature sensor 32 is positioned close to the second battery 12, for example adjacent to the second battery 12. The temperature detected by the second temperature sensor 32 may be deemed as the temperature of the entire inhaler device 100.

The CPU 40 transmits or receives control signals to or from the structural elements included in the inhaler device 100 to control the structural elements. In one example, the CPU 40 controls the operation of the heater 121 by turning on or off the FET 22. In another example, the CPU 40 controls charging of the first battery 11 and the second battery 12 by controlling the first charger IC 13 and the second charger IC 14. The CPU 40 also controls power supply from the external power supply 200 to the inhaler device 100 by transmitting or receiving control signals to or from the external power supply 200 coupled to the inhaler device 100.

As illustrated in Fig. 3, the first battery 11 is operable to supply electric power to the second battery 12. The CPU 40 may thus control the second battery 12 to supply electric power to the first battery 11 to charge the first battery 11. The power consumption of the heater 121 is higher than the power consumption of the CPU 40, and thus, the residual capacity of the first battery 11 can become low earlier than the second battery 12. In this regard, with this configuration, when the residual capacity of the first battery 11 is low, electric power of the second battery 12 can be supplied to the heater 121 via the first battery 11, so that heating by the heater 121 is continuously provided.

Fig. 3 illustrates the example in which the first battery 11 is coupled to the heater 121, but the second battery 12 may also be coupled to the heater 121. For example, the second battery 12 may be coupled to the heater 121 via the protection element 21 and the FET 22. In this case, the second battery 12 is operable to supply electric power to the heater 121. Such a configuration is often seen as a common configuration including one battery; although not illustrated in the drawings, this configuration may be used. The CPU 40 may control the first battery 11 or the second battery 12 to supply electric power to the heater 121. This configuration enables a most suitable power source to the circumstances to supply electric power to the heater 121, which ensures the most effective use of the battery.

More specifically, the CPU 40 may control the second battery 12 to supply electric power to the CPU 40 and control the first battery 11 or the second battery 12 to supply electric power to the heater 121. For example, in the normal state, the CPU 40 controls the first battery 11 to supply electric power to the heater 121. In particular circumstances, the CPU 40 provides switching control to cause the second battery 12 to supply electric power to the heater 121. This configuration enables a most suitable power source to the circumstances to supply electric power to the heater 121. The following describes an example of the switching control.

The CPU 40 may control the first battery 11 or the second battery 12 to supply electric power to the heater 121 depending on the temperature sensed by the first temperature sensor 31. Specifically, when the temperature sensed by the first temperature sensor 31 satisfies a predetermined condition, the CPU 40 may control the first battery 11 to supply electric power to the heater 121. By contrast, when the temperature sensed by the first temperature sensor 31 fails to satisfy the predetermined condition, the CPU 40 may control the second battery 12 to supply electric power to the heater 121. An example of the predetermined condition is that the temperature sensed by the first temperature sensor 31 is smaller than or equal to a threshold. An example of the threshold is a low temperature, for example 0°C. In this case, in low-temperature environments, the first battery 11 that is an all-solid-state battery having high operational tolerance to low temperatures is designated to supply electric power to the heater 121. Another example of the threshold is a high temperature that exceeds an operating limit that is set in a system. For example, after heating has been continuously performed, it is assumed that under the effect of remaining heat, the temperature of the portion around the heater can exceed the usage temperature limit of the first battery 11 that is set in the system. Under such excessively high temperatures, it is desirable to avoid the use of the first battery 11. In response, the temperature of the second battery 12, which is almost the same as the temperature of the entire device because the second battery 12 is positioned away from the heater 121, is checked; when the temperature of the second battery 12 is an operable temperature, the second battery 12 is designated for use. As such, the safety is secured.

The CPU 40 may provide switching control to cause the first battery 11 or the second battery 12 to supply electric power to the heater 121 depending on the residual capacity of the first battery 11. Specifically, when the residual capacity of the first battery 11 is greater than or equal to the threshold, the CPU 40 may control the first battery 11 to supply electric power to the heater 121. By contrast, when the residual capacity of the first battery 11 is smaller than the threshold, the CPU 40 may control the second battery 12 to supply electric power to the heater 121. This configuration enables the heater 121 to continue heating after the residual capacity of the first battery 11 becomes low.

When the inhaler device 100 is coupled to the external power supply 200, the CPU 40 may control the first battery 11 and the second battery 12 to be charged. As well as performing the control operation described above in the normal state, the CPU 40 may perform other kinds of control operation depending on the circumstances.

For example, when the inhaler device 100 is coupled to the external power supply 200, the CPU 40 may provide switching control to cause only the first battery 11 or both of the first battery 11 and the second battery 12 to be charged, depending on the temperature sensed by the first temperature sensor 31. Specifically, when the temperature sensed by the first temperature sensor 31 is excessively low temperatures, the CPU 40 may control only the first battery 11 to be charged. By contrast, when the temperature sensed by the first temperature sensor 31 is not excessively low temperatures, the CPU 40 controls both of the first battery 11 and the second battery 12 to be charged. An example of the excessively low temperature is 0°C or lower. In this case, in low-temperature environments, priority for charging is given to the first battery 11 that is an all-solid-state battery having high operational tolerance to low temperatures.

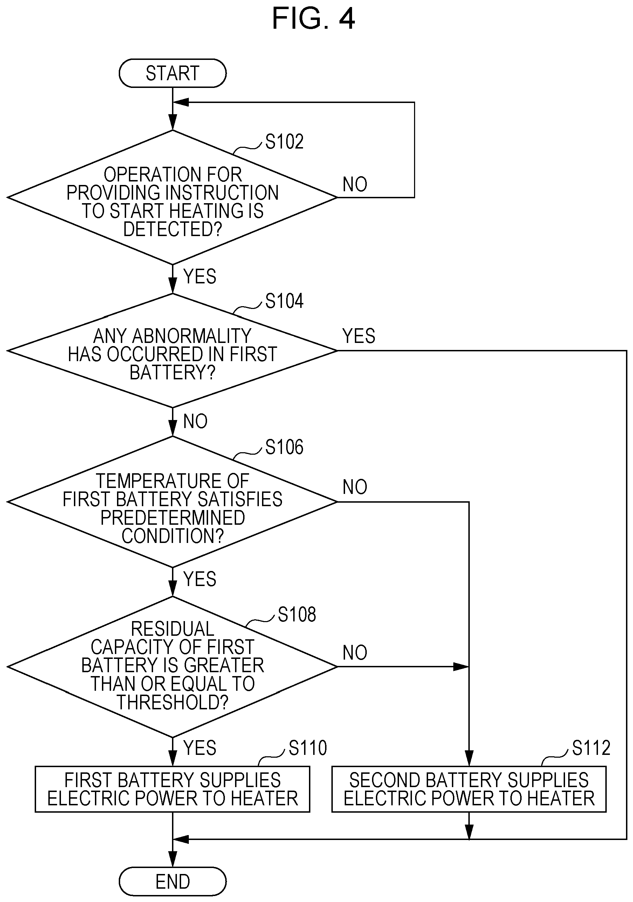

The following describes an example of a process flow implemented by the inhaler device 100 according to the present embodiment with reference to Fig. 4. Fig. 4 is a flowchart illustrating an example of a process flow implemented by the inhaler device 100 according to the present embodiment.

As illustrated in FIG. 4, firstly, the CPU 40 determines whether an operation for providing an instruction to start heating is detected (step S102). An example of the operation for providing an instruction to start heating is to press a button provided on the inhaler device 100. Another example of the operation for providing an instruction to start heating is to perform a puff action. Still another example of the operation for providing an instruction to start heating is to receive a signal from another device such as a smartphone. The CPU 40 waits until the operation for providing an instruction to start heating is detected (NO in step S102).

When the operation for providing an instruction to start heating is detected (YES in step S102), the CPU 40 determines whether any abnormality has occurred in the first battery 11 (step S104). When it is determined that any abnormality has occurred in the first battery 11 (YES in step S104), the process ends.

When it is determined that no abnormality has occurred in the first battery 11 (NO in step S104), the CPU 40 determines whether the temperature of the first battery 11 satisfies the predetermined condition (step S106). When it is determined that the temperature of the first battery 11 does not satisfy the predetermined condition (NO in step S106), the process proceeds to step S112.

When it is determined that the temperature of the first battery 11 satisfies the predetermined condition (YES in step S106), the CPU 40 determines whether the residual capacity of the first battery 11 is greater than or equal to a threshold (step S108). When it is determined that the residual capacity of the first battery 11 is smaller than the threshold (NO in step S108), the process proceeds to step S112.

When it is determined that the residual capacity of the first battery 11 is greater than or equal to the threshold (YES in step S108), the CPU 40 controls the first battery 11 to supply electric power to the heater 121 (step S110). In response to this, heating by the heater 121 starts.

In step S112, the CPU 40 controls the second battery 12 to supply electric power to the heater 121. In response to this, heating by the heater 121 starts.

### 3. Supplement

A preferred embodiment of the present invention has been described in detail with reference to the accompanying drawings, but the present invention is not limited to this example. It is understood that various modifications and alterations within the scope of the technical idea indicated in the appended claims may occur to those skilled in the art and are also embraced within the technical scope of the present invention.

For example, in the embodiment described above, the example in which while controlling the second battery 12 to supply electric power to the CPU 40, the CPU 40 provides switching control to cause the first battery 11 or the second battery 12 to supply electric power to the heater 121 depending on the circumstances has been described. However, the present invention is not limited to this example. In one example, while controlling the first battery 11 to supply electric power to the heater 121, the CPU 40 may provide switching control to cause the first battery 11 or the second battery 12 to supply electric power to the CPU 40 depending on the circumstances. This configuration enables a most suitable power source to the circumstances to supply electric power to the CPU 40.

The series of operations performed by the device described in this specification may be implemented by software, hardware, or a combination of software and hardware. A program as software is previously stored, for example, inside the device or in external storage media (non-transitory media). For example, when a computer for controlling the device described in this specification runs the program, the program is loaded on a random-access memory (RAM) and executed by a processor such as a CPU. The storage medium may be, for example, a magnetic disk, optical disk, magneto-optical disk, or flash memory. Instead of using the storage medium, the computer program described above may be, for example, delivered through a network.

The operations described using a flowchart in this specification are not necessarily performed in the order illustrated. Some of the operational steps may be parallelly performed. Alternatively, additional operational steps may be used, or some operational steps may be removed.

### Reference Signs List

100 inhaler device
110 power supply unit
111 power supply
112 sensor
113 notifier
114 memory
115 communicator
116 controller
120 cartridge
121 heater
122 liquid guide
123 liquid storage
124 mouthpiece
130 flavor imparting cartridge
131 flavor source
140 holder
141 internal space
142 opening
143 bottom
144 heat insulator
150 stick substrate
151 substrate
152 inhalation port
180 airflow path
181 air inlet hole
182 air outlet hole
11 first battery
12 second battery
13 first charger IC
14 second charger IC
21 protection element
22 FET
23 LDO
31 first temperature sensor
32 second temperature sensor
40 CPU
200 external power supply

## Claims

1. An inhaler device (100) comprising:
a generator (121) configured to generate an aerosol using a substrate containing an aerosol source,
a controller (40) configured to control operation of the generator (121), and
a first battery (11) and a second battery (12) that are discrete, wherein
the controller (40) is configured to control the first battery (11) or the second battery (12) to supply electric power to the generator (121), and
the first battery (11) is operable to supply electric power to the generator (121), and the second battery (12) is operable to supply electric power to the controller (40) and the generator (121).

2. The inhaler device (100) according to claim 1, wherein
the controller (40) is configured to control the second battery (12) to supply electric power to the controller (40) and also control the first battery (11) or the second battery (12) to supply electric power to the generator (121).

3. The inhaler device (100) according to any one of claims 1 to 2, wherein
the first battery (11) is an all-solid-state battery.

4. The inhaler device (100) according to any one of claims 1 to 3, wherein
the first battery (11) is positioned close to the generator (121).

5. The inhaler device (100) according to claim 4, further comprising:
a temperature sensor configured to sense a temperature of the first battery (11) and a temperature of the generator (121).

6. The inhaler device (100) according to claim 5, wherein
the controller (40) is configured to, depending on the temperature sensed by the temperature sensor, control the first battery (11) or the second battery (12) to supply electric power to the generator (121).

7. The inhaler device (100) according to claim 6, wherein
the controller (40) is configured to, when the temperature sensed by the temperature sensor satisfies a predetermined condition, control the first battery (11) to supply electric power to the generator (121).

8. The inhaler device (100) according to any one of claims 5 to 7, wherein
the controller (40) is configured to, when the inhaler device is coupled to an external power supply, provide switching control to cause only the first battery (11) or both of the first battery (11) and the second battery (12) to be charged, depending on the temperature sensed by the temperature sensor.

9. The inhaler device (100) according to any one of claims 1 to 7, wherein
the controller (40) is configured to, when the inhaler device is coupled to an external power supply, cause the first battery (11) and the second battery (12) to be charged.

10. The inhaler device (100) according to any one of claims 1 to 9, wherein
the controller (40) is configured to, depending on a residual capacity of the first battery (11), provide switching control to cause the first battery (11) or the second battery (12) to supply electric power to the generator (121).

11. The inhaler device (100) according to any one of claims 1 to 10, wherein
the controller (40) is configured to control the second battery (12) to supply electric power to the first battery to charge the first battery (11).

12. The inhaler device (100) according to any one of claims 1 to 11, further comprising:
a protection element configured to stop overcurrent, wherein
the protection element is provided in a circuit coupling the first battery (11) and the generator (121).

13. A control method for controlling an inhaler device (100),
the inhaler device (100) including
a generator (121) configured to generate an aerosol using a substrate containing an aerosol source,
a controller (40) configured to control operation of the generator (121); and
a first battery (11) and a second battery (12) that are discrete, wherein
the first battery (11) is operable to supply electric power to the generator (121), and the second battery (12) is operable to supply electric power to the controller (40) and the generator (121);
the control method comprising: with respect to each of the generator (121) and the controller (40), controlling the first battery (11) or the second battery (12) to supply electric power.

14. A program configured to be run by a computer for controlling an inhaler device (100),
the inhaler device (100) including
a generator (121) configured to generate an aerosol using a substrate containing an aerosol source,
a controller (40) configured to control operation of the generator (121); and
a first battery (11) and a second battery (12) that are discrete, wherein
the first battery (11) is operable to supply electric power to the generator, and the second battery (12) is operable to supply electric power to the controller (40) and the generator (121);
the program causing the computer to execute a process comprising:
with respect to each of the generator (121) and the controller (40), controlling the first battery (11) or the second battery (12) to supply electric power.

## Patentansprüche

1. Inhalationsvorrichtung (100), umfassend:
eine Erzeugungseinheit (121), die dazu ausgebildet ist, ein Aerosol unter Verwendung eines Substrats zu erzeugen, das eine Aerosolquelle enthält;
eine Steuereinheit (40), die dazu ausgebildet ist, den Betrieb der Erzeugungseinheit (121) zu steuern; und
eine erste Batterie (11) und eine zweite Batterie (12), die voneinander getrennt sind, wobei
die Steuereinheit (40) dazu ausgebildet ist, die erste Batterie (11) oder die zweite Batterie (12) zur Versorgung der Erzeugungseinheit (121) mit elektrischer Leistung zu steuern, und
die erste Batterie (11) betreibbar ist, um die Erzeugungseinheit (121) mit elektrischer Leistung zu versorgen, und die zweite Batterie (12) betreibbar ist, um die Steuereinheit (40) und die Erzeugungseinheit (121) mit elektrischer Leistung zu versorgen.

2. Inhalationsvorrichtung (100) nach Anspruch 1, wobei
die Steuereinheit (40) dazu ausgebildet ist, die zweite Batterie (12) zur Versorgung der Steuereinheit (40) mit elektrischer Leistung zu steuern und weiter die erste Batterie (11) oder die zweite Batterie (12) zur Versorgung der Erzeugungseinheit (121) mit elektrischer Leistung zu steuern.

3. Inhalationsvorrichtung (100) nach einem der Ansprüche 1 bis 2, wobei
die erste Batterie (11) eine Festkörperbatterie ist.

4. Inhalationsvorrichtung (100) nach einem der Ansprüche 1 bis 3, wobei die erste Batterie (11) nahe bei der Erzeugungseinheit (121) positioniert ist.

5. Inhalationsvorrichtung (100) nach Anspruch 4, weiter umfassend:
einen Temperatursensor, der dazu ausgebildet ist, eine Temperatur der ersten Batterie (11) und eine Temperatur der Erzeugungseinheit (121) zu erfassen.

6. Inhalationsvorrichtung (100) nach Anspruch 5, wobei
die Steuereinheit (40) dazu ausgebildet ist, abhängig von der von dem Temperatursensor erfassten Temperatur die erste Batterie (11) oder die zweite Batterie (12) zur Versorgung der Erzeugungseinheit (121) mit elektrischer Leistung zu steuern.

7. Inhalationsvorrichtung (100) nach Anspruch 6, wobei
die Steuereinheit (40) dazu ausgebildet ist, wenn die von dem Temperatursensor erfasste Temperatur eine vorbestimmte Bedingung erfüllt, die erste Batterie (11) so zu steuern, dass sie die Erzeugungseinheit (121) mit elektrischer Leistung versorgt.

8. Inhalationsvorrichtung (100) nach einem der Ansprüche 5 bis 7, wobei
die Steuereinheit (40) dazu ausgebildet ist, wenn die Inhalationsvorrichtung an eine externe Stromversorgung gekoppelt ist, abhängig von der von dem Temperatursensor erfassten Temperatur eine Umschaltsteuerung bereitzustellen, um zu bewirken, dass nur die erste Batterie (11) oder sowohl die erste Batterie (11) als auch die zweite Batterie (12) geladen werden.

9. Inhalationsvorrichtung (100) nach einem der Ansprüche 1 bis 7, wobei
die Steuereinheit (40) dazu ausgebildet ist, wenn die Inhalationsvorrichtung an eine externe Stromversorgung gekoppelt ist, zu bewirken, dass die erste Batterie (11) und die zweite Batterie (12) geladen werden.

10. Inhalationsvorrichtung (100) nach einem der Ansprüche 1 bis 9, wobei
die Steuereinheit (40) dazu ausgebildet ist, abhängig von einer Restkapazität der ersten Batterie (11) eine Umschaltsteuerung bereitzustellen, um zu bewirken, dass die erste Batterie (11) oder die zweite Batterie (12) die Erzeugungseinheit (121) mit elektrischer Leistung versorgt.

11. Inhalationsvorrichtung (100) nach einem der Ansprüche 1 bis 10, wobei
die Steuereinheit (40) dazu ausgebildet ist, die zweite Batterie (12) so zu steuern, dass sie die erste Batterie mit elektrischer Leistung versorgt, um die erste Batterie (11) zu laden.

12. Inhalationsvorrichtung (100) nach einem der Ansprüche 1 bis 11, weiter umfassend:
ein Schutzelement, das dazu ausgebildet ist, einen Überstrom zu stoppen, wobei
das Schutzelement in einer Schaltung bereitgestellt ist, die die erste Batterie (11) und die Erzeugungseinheit (121) koppelt.

13. Steuerungsverfahren zum Steuern einer Inhalationsvorrichtung (100),
wobei die Inhalationsvorrichtung (100) Folgendes einschließt
eine Erzeugungseinheit (121), die dazu ausgebildet ist, ein Aerosol unter Verwendung von einem Substrat zu erzeugen, das eine Aerosolquelle enthält,
eine Steuereinheit (40), die dazu ausgebildet ist, den Betrieb der Erzeugungseinheit (121) zu steuern; und
eine erste Batterie (11) und eine zweite Batterie (12), die voneinander getrennt sind, wobei
die erste Batterie (11) betreibbar ist, um die Erzeugungseinheit (121) mit elektrischer Leistung zu versorgen, und die zweite Batterie (12) betreibbar ist, um die Steuereinheit (40) und die Erzeugungseinheit (121) mit elektrischer Leistung zu versorgen;
wobei das Steuerungsverfahren Folgendes umfasst:
in Bezug auf jede der Erzeugungseinheit (121) und der Steuereinheit (40), die erste Batterie (11) oder die zweite Batterie (12) jeweils zur Versorgung mit elektrischer Leistung zu steuern.

14. Programm, das dazu ausgebildet ist, von einem Computer zum Steuern einer Inhalationsvorrichtung (100) ausgeführt zu werden,
wobei die Inhalationsvorrichtung (100) Folgendes einschließt
eine Erzeugungseinheit (121), die dazu ausgebildet ist, ein Aerosol unter Verwendung eines Substrats zu erzeugen, das eine Aerosolquelle enthält,
eine Steuereinheit (40), die dazu ausgebildet ist, den Betrieb der Erzeugungseinheit (121) zu steuern; und
eine erste Batterie (11) und eine zweite Batterie (12), die voneinander getrennt sind, wobei
die erste Batterie (11) betreibbar ist, um die Erzeugungseinheit mit elektrischer Leistung zu versorgen, und die zweite Batterie (12) betreibbar ist, um die Steuereinheit (40) und die Erzeugungseinheit (121) mit elektrischer Leistung zu versorgen;
wobei das Programm bewirkt, dass der Computer einen Prozess ausführt, umfassend:
in Bezug auf jede der Erzeugungseinheit (121) und der Steuereinheit (40), die erste Batterie (11) oder die zweite Batterie (12) jeweils zur Versorgung mit elektrischer Leistung zu steuern.

## Revendications

1. Dispositif d'inhalation (100) comprenant :
un générateur (121) configuré pour générer un aérosol en utilisant un substrat contenant une source d'aérosol ;
une unité de commande (40) configurée pour commander le fonctionnement du générateur (121) ; et
une première batterie (11) et une seconde batterie (12) qui sont distinctes, dans lequel
l'unité de commande (40) est configurée pour commander la première batterie (11) ou la seconde batterie (12) afin de fournir de l'énergie électrique au générateur (121), et
la première batterie (11) est apte à fournir de l'énergie électrique au générateur (121), et la seconde batterie (12) est apte à fournir de l'énergie électrique à l'unité de commande (40) et au générateur (121).

2. Dispositif d'inhalation (100) selon la revendication 1, dans lequel
l'unité de commande (40) est configurée pour commander la seconde batterie (12) afin de fournir de l'énergie électrique à l'unité de commande (40) et commander également la première batterie (11) ou la seconde batterie (12) afin de fournir de l'énergie électrique au générateur (121).

3. Dispositif d'inhalation (100) selon l'une quelconque des revendications 1 à 2, dans lequel
la première batterie (11) est une batterie à l'état solide.

4. Dispositif d'inhalation (100) selon l'une quelconque des revendications 1 à 3, dans lequel
la première batterie (11) est positionnée à proximité du générateur (121).

5. Dispositif d'inhalation (100) selon la revendication 4, comprenant en outre :
un capteur de température configuré pour détecter une température de la première batterie (11) et une température du générateur (121).

6. Dispositif d'inhalation (100) selon la revendication 5, dans lequel
l'unité de commande (40) est configurée pour, en fonction de la température détectée par le capteur de température, commander la première batterie (11) ou la seconde batterie (12) afin de fournir de l'énergie électrique au générateur (121).

7. Dispositif d'inhalation (100) selon la revendication 6, dans lequel
l'unité de commande (40) est configurée pour, lorsque la température détectée par le capteur de température satisfait une condition prédéterminée, commander la première batterie (11) afin de fournir de l'énergie électrique au générateur (121).

8. Dispositif d'inhalation (100) selon l'une quelconque des revendications 5 à 7, dans lequel
l'unité de commande (40) est configurée pour, lorsque le dispositif d'inhalation est couplé à une alimentation électrique externe, effectuer une commande de commutation de manière à amener seulement la première batterie (11) ou à la fois la première batterie (11) et la seconde batterie (12) à être chargées, en fonction de la température détectée par le capteur de température.

9. Dispositif d'inhalation (100) selon l'une quelconque des revendications 1 à 7, dans lequel
l'unité de commande (40) est configurée pour, lorsque le dispositif d'inhalation est couplé à une alimentation électrique externe, amener la première batterie (11) et la seconde batterie (12) à être chargées.

10. Dispositif d'inhalation (100) selon l'une quelconque des revendications 1 à 9, dans lequel
l'unité de commande (40) est configurée pour, en fonction d'une capacité résiduelle de la première batterie (11), effectuer une commande de commutation de manière à amener la première batterie (11) ou la seconde batterie (12) à fournir de l'énergie électrique au générateur (121).

11. Dispositif d'inhalation (100) selon l'une quelconque des revendications 1 à 10, dans lequel
l'unité de commande (40) est configurée pour commander la seconde batterie (12) afin de fournir de l'énergie électrique à la première batterie afin de charger la première batterie (11).

12. Dispositif d'inhalation (100) selon l'une quelconque des revendications 1 à 11, comprenant en outre :
un élément de protection configuré pour arrêter une surintensité, dans lequel
l'élément de protection est prévu dans un circuit couplant la première batterie (11) et le générateur (121).

13. Procédé de commande pour commander un dispositif d'inhalation (100),
le dispositif d'inhalation (100) incluant
un générateur (121) configuré pour générer un aérosol en utilisant un substrat contenant une source d'aérosol,
une unité de commande (40) configurée pour commander le fonctionnement du générateur (121) ; et
une première batterie (11) et une seconde batterie (12) qui sont distinctes, dans lequel
la première batterie (11) est apte à fournir de l'énergie électrique au générateur (121), et la seconde batterie (12) est apte à fournir de l'énergie électrique à l'unité de commande (40) et au générateur (121) ;
le procédé de commande comprenant :
en ce qui concerne chacun du générateur (121) et de l'unité de commande (40), commander la première batterie (11) ou la seconde batterie (12) afin de fournir de l'énergie électrique.

14. Programme configuré pour être exécuté par un ordinateur pour commander un dispositif d'inhalation (100),
le dispositif d'inhalation (100) incluant
un générateur (121) configuré pour générer un aérosol en utilisant un substrat contenant une source d'aérosol,
une unité de commande (40) configurée pour commander le fonctionnement du générateur (121) ; et
une première batterie (11) et une seconde batterie (12) qui sont distinctes, dans lequel
la première batterie (11) est apte à fournir de l'énergie électrique au générateur, et la seconde batterie (12) est apte à fournir de l'énergie électrique à l'unité de commande (40) et au générateur (121) ;
le programme amenant l'ordinateur à exécuter un procédé comprenant :
en ce qui concerne chacun du générateur (121) et de l'unité de commande (40), commander la première batterie (11) ou la seconde batterie (12) afin de fournir de l'énergie électrique.
